# EUROPEAN PATENT SPECIFICATION

(11) **EP 3 288 531 B1**
(45) Date of publication and mention of the grant of the patent: **24.07.2019**
(21) Application number: 16725638.7
(22) Date of filing: 29.04.2016
(51) Int. Cl.: A61K 8/67, A61Q 19/08

(54) **METHOD OF IMPROVING SKIN HEALTH AND COMPOSITIONS THEREFOR**
METHODE ZUR VERBESSERUNG DER HAUTGESUNDHEIT UND ZUSAMMENSETZUNGEN DAFÜR
METHODE D'AMELIORATION DE LA SANTE DE LA PEAU ET COMPOSITIONS POUR

(30) Priority: 01.05.2015 US 201562155722 P
(43) Date of publication of application: 07.03.2018
(73) Proprietor: The Procter & Gamble Company, Cincinnati, OH 45202 (US)
(72) Inventor: HAKOZAKI, Tomohiro, Cincinnati, Ohio 45202 (US); LAUGHLIN, Leo, Timothy, II, Cincinnati, Ohio 45202 (US); VELAZQUEZ, Jesus, Cincinnati, Ohio 45202 (US); OBLONG, John, Erich, Cincinnati, Ohio 45202 (US); MILLS, Kevin, John, Cincinnati, Ohio 45202 (US)
(74) Representative: Engisch, Gautier
(86) International application number: PCT/US2016/029951
(87) International publication number: WO 2016/178949

(56) References cited:
- WO-A2-01/51051
- WO-A2-2006/105440
- US-A1- 2007 117 765

## Description

### FIELD OF THE INVENTION

The present disclosure relates generally to cosmetic methods and compositions for improving the appearance of skin using a synergistic combination of niacinamide and nicotinamide riboside to mitigate the effects of reactive oxygen species on cellular function.

### BACKGROUND OF THE INVENTION

The impact of various environmental stressors such as ultraviolet radiation, heat, smog and cigarette smoke are known to cause oxidative stress to skin cells. According to some theories, these environment stressors create highly reactive molecules (e.g., free radicals and exogenous reactive oxygen species ("ROS")) within skin cells that damage various cellular organelles, structural membranes, lipids, proteins and nucleic acids. A reactive oxygen species such as a hydroxyl radical is extremely reactive and will remove electrons from virtually any molecule in its path, turning that molecule into a free radical and thus propagating a chain reaction. However, some ROS such as hydrogen peroxide, which are generally less reactive than a hydroxyl radical, can actually be more damaging to DNA than the hydroxyl radical, since the lower reactivity of hydrogen peroxide provides enough time for the molecule to travel into the nucleus of the cell where it can damage DNA. While exogenous ROS are notorious for causing oxidative stress, even naturally occurring biological processes such as cellular metabolism are known to form endogenous ROS. Fortunately, most skin cells are equipped with an antioxidant defense system, which seeks to maintain a reducing environment in the cell by providing a variety of redox regulators such as glutathione and nicotinamide adenine dinucleotide ("NAD") to neutralize endogenous and exogenous ROS.

A healthy antioxidant defense system is usually adequate for managing the harmful effects of ROS produced from cellular metabolism and the other naturally occurring cellular functions (i.e., endogenous ROS). But when a skin cell is exposed to an external stressor such as heat or UV radiation, ROS levels can increase dramatically. Oxidative stress occurs from cumulative damage caused by ROS over time, and eventually can lead to tissue damage and/or organ dysfunction. For example, skin cells may manifest accumulated oxidative stress as noticeable changes in the skin's structure and morphology (e.g., hyperpigmentation, sallowness, wrinkles, lines, reduced skin thickness, loss of elasticity) and, to a more extreme degree, skin carcinomas. In some instances, accumulated oxidative stress to organelles such as mitochondria may lead to reduced levels of ATP and/or NAD, which can result in premature aging of skin.

The antioxidant defense system of most cells is typically controlled by a master switch referred to as the antioxidant response element ("ARE"). The ARE is a cis-acting enhancer sequence located in the regulatory regions of antioxidant and detoxifying genes, and is activated by redox-cycling phenols and electrophiles. Some studies suggest that the DNA-binding protein Nuclear Factor-Like 2 ("Nrf2") is the principal transcription factor necessary for ARE activation, even though many other transcription factors bind to the ARE sequence. When the ARE is activated in response to oxidative stress, the corresponding genes signal the cell to begin producing reduction/oxidation regulators and/or ROS quenching proteins and enzymes. In addition to modulating the production of redox regulators and ROS quenching compounds, the ARE can also signal the cell to begin repair. Some of the biochemical pathways associated with the human ARE have been well elucidated, while others have not. Accordingly, it would be desirable to modulate the ARE of skin cells to mitigate the damage cause by oxidative stress to skin.

U.S. Patent No. 8,106,184 ("Sauve") is directed to utilizing nicotinoyl ribosides and nicotinamide riboside for increasing intracellular levels of nicotinamide adenine dinucleotide (NAD+) in cells and tissues. Sauve discloses that skin can be protected from aging (e.g., developing wrinkles, loss of elasticity, etc.) by treating skin or epithelial cells with a nicotinoyl riboside or derivative compound that increases the level intracellular NAD+. Sauve also discloses that exemplary skin afflictions or skin conditions treatable by its methods include disorders or diseases associated with or caused by inflammation, sun damage or natural aging. However, Sauve only discusses the effect of nicotinoyl riboside on the NAD+ metabolic pathway to the exclusion of other biological pathways. Thus, there may still be other biological pathways related to oxidative stress that can be exploited to mitigate the detrimental effects of environment stressors on cellular health and metabolism.

U.S. Publication No. 2005/0267023 ("Sinclair") is directed to methods and compositions for modulating the life span of a cell or its resistance to stress, comprising modulating the flux through the NAD+ salvage pathway in the cell, which may comprise increasing the level or activity of a protein selected from the group consisting of NPT1, PNC1, NMA1 and NMA2. Sinclair discloses an embodiment wherein the amount and/or activity of nicotinamide (a.k.a. niacinamide) is reduced. Sinclair also discloses an embodiment wherein the NAD+ salvage pathway in a cell is stimulated by contacting the cell with nicotinamide riboside, or a biologically active analog and/or prodrug thereof. Increasing the lifespan of a cell or the resistance of a cell to stress is important for the overall health of the cell and/or tissue, but there still remains a need to identify effective methods and actives for mitigating the effects of oxidative stress on cells.

U.S. Publication No. 2011/0262570 ("Finlay") relates to gene panels, microarrays and biomarker panels that include genes and gene products associated with age-related oxidative damage to skin, and transcriptional profiling-based methods for identification and evaluation of cosmetic agents for prevention, reversal, or reduction of oxidative damage to skin. In particular, Finlay seeks to identify and/or evaluate cosmetic agents capable of inducing nrf2-mediated activation of the antioxidant response element to increase expression of Phase 2 enzymes. Finlay discloses that the cosmetic industry is in need of antioxidant agents and cosmetic formulations comprising them that act through mechanisms other than direct scavenging. Finlay discloses some examples of general classes of materials that may be capable of inducing nrf2-mediated activation of the antioxidant response element, and a few specific examples. But there is still a need to identify skin care agents, which are capable of activating the ARE alone or in combination with other agents.

U.S. Publication No. 2011/0262025 ("Jarrold") relates to gene panels, biomarker panels and microarrays relating to lipid formation in stratum corneum of human skin as a function of extrinsic and/or intrinsic aging conditions Jarrold discloses that a greater understanding of the biochemical processes responsible for aging has invigorated the cosmetics industry and resulted in the emergence of a new class of cosmetic actives which are purported to be antioxidants, antiinflammatories and free-radical-scavengers. Jarrold discloses that *Galactomyces* ferment filtrate has been shown to have antioxidant effects on human skin through activation of ARE-related genes in human skin cells and induction of hyaluronan production in epidermal cells.

PCT Publication No. WO2015/009879 ("Pi") relates to compounds that can inhibit Nrf2 activity, and more particularly Nrf2-ARE activity. Pi also relates to pharmaceutical compositions containing such compounds. Pi discloses a desire to inhibit Nrf2 activation of the ARE to prevent certain diseases such as hepatitis and cancer from benefitting from the antioxidant protection provided by Nrf2 activation of the ARE. Pi discloses a list of exemplary actives for inhibiting Nrf2-ARE activity, which include isonicotinamide (an isomer of niacinamide) and 2-amino- isonicotinamide. However, the actives in Pi may not be suitable in cosmetic compositions, especially compositions that are intended to promote ARE activity rather than inhibiting it.

PCT Publication No. WO 2014/151891 ("Walley") relates to topical products comprising a plurality of Nrf2-activating agents, which are effective for reducing oxidative stress and improving conditions associated with oxidative stress in hard surfaces such as skin, hair and/or nails of a subject. Walley discloses examples of various agents, many of which are botanical extracts. However, there is still a need to identify improved actives that mitigate the damage caused by oxidative stress to skin cells.

U.S. Serial No. 14/050183 ("Oblong") relates to identifying and/or evaluating actives that reduce, prevent and/or reverse the undesirable oxidative stress on a particular type of skin cell that is associated with a particular stressor. In particular, Oblong expresses an interest in skin care actives and skin cells. Oblong discloses cosmetic compositions that may include a dermatologically acceptable carrier and a skin-care active such as niacinamide, which reduces, prevents and/or reverses the undesirable metabolic effects of oxidative stress on skin cells. Oblong discloses that niacinamide does not affect glycolysis metabolism but lessens the detrimental effect of hydrogen peroxide on fibroblast glycolysis metabolism.

Accordingly, it would be desirable to identify cosmetic skin care actives that provide improved ARE activation capability. It would also be desirable to identify combinations of cosmetic skin care actives that provide synergistic ARE activation capability. It would further be desirable to identify cosmetic skin care actives that mitigate the detrimental effects of ROS on cellular metabolism. It would still further be desirable to identify combinations of cosmetic skin care actives that synergistically mitigate the detrimental effects of ROS on cellular metabolism.

WO 01/51051 discloses the use of niacinamide in cosmetic methods.

WO 2006/105440 discloses the use of nicotinamide riboside for treating skin.

US 2007/117765 discloses the use of nicotinamide riboside for increasing intracellular NAD levels.

### BRIEF DESCRIPTION OF THE DRAWINGS

FIG. 1 illustrates the glycolysis metabolic pathway.
FIG. 2 illustrates a portion of the NAD+ pathway.
FIG. 3 is a chart illustrating ARE activation by niacinamide and nicotinamide riboside.
FIG. 4 is a chart illustrating glycolytic ATP levels as determined by the ECAR Assay.
FIG. 5 is a chart illustrating glycolytic ATP depletion caused by hydrogen peroxide.

### SUMMARY OF THE INVENTION

The present disclosure relates to a cosmetic method of activating gene transcription through the antioxidant response element (ARE) of a skin cell, comprising: identifying a target portion of skin comprising skin cells where increased ARE activated gene transcription through is desired and/or needed; and contacting at least some of the skin cells with an effective amount of niacinamide and nicotinamide riboside in combination for a treatment period sufficient for the combination of niacinamide and nicotinamide riboside to increase ARE activated gene transcription of at least some of the contacted skin cells contacted Also disclosed is a cosmetic method of synergistically reducing ROS-induced adenosine triphosphate (ATP) depletion in a skin cell, comprising: identifying a target portion of skin comprising skin cells where reduced ROS-induced ATP depletion is desired and/or needed; and contacting at least some of the skin cells with an effective amount of niacinamide and nicotinamide riboside in combination for a treatment period sufficient for the effective amount of niacinamide and nicotinamide riboside to synergistically reduce the ATP depletion in at least some of the contacted skin cells. Further disclosed is a cosmetic method of restoring glycolytic ATP production rate in a skin cell that has been reduced as a result of ROS-induced oxidative stress: comrpsing identifying a target portion of skin comprising skin cells where restored glycolytic ATP production rate reduced as a result of ROS-induced oxidative stress is desired and/or needed; and contacting at least some of the skin cells with an effective amount of niacinamide and nicotinamide riboside in combination for a treatment period sufficient for the effective amount of niacinamide and nicotinamide riboside to synergistically restore the glycolytic ATP production rate in at least some of the contacted skin cells.

### DETAILED DESCRIPTION OF THE INVENTION

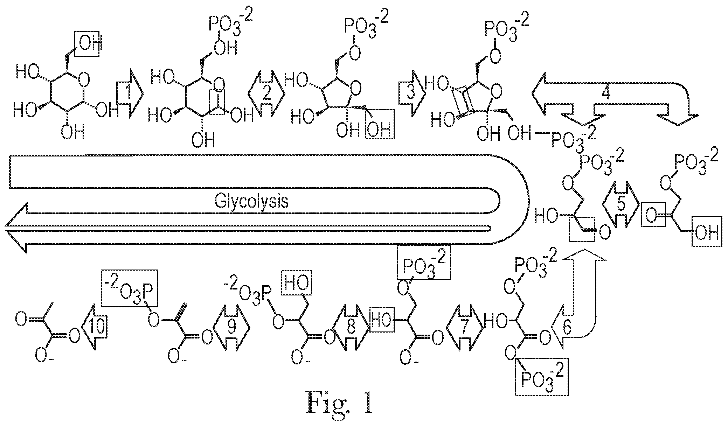

As humans age, oxidative damage from external and internal stressors on the cells of the body accumulates, which may lead to decreased efficiency and function of tissue and organs (i.e., oxidative stress). It is not uncommon for oxidative stress to manifest as a reduction in the ability of the cells to produce energy and/or a change in the functioning of the cells. Prior to the present invention, it was unknown whether nicotinamide riboside and/or niacinamide would increase ARE activated gene transcription. It was also unknown whether nicotinamide riboside has any effect on the glycolysis metabolic pathway, which is illustrated in FIG. 1, or is able to reduce ATP depletion associated with ROS-induced oxidative stress.

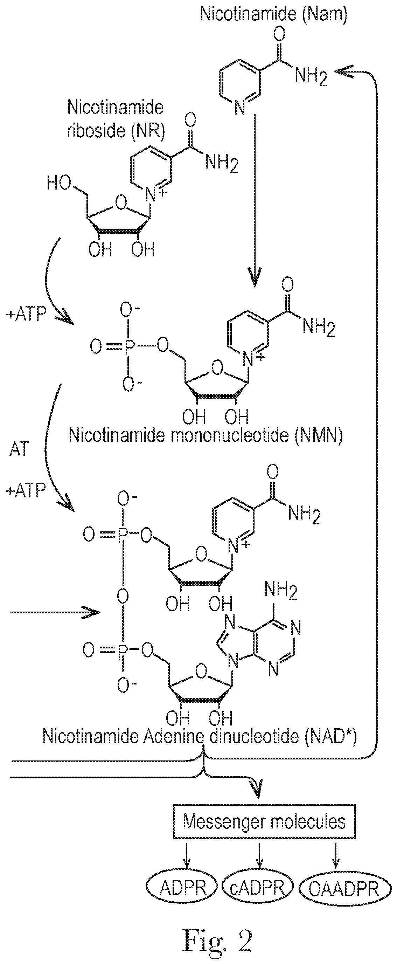

Now it has been discovered that nicotinamide riboside is capable of increasing ARE activated gene transcription and restoring glycolytic ATP production rates. Surprisingly, it has also been discovered that niacinamide and nicotinamide riboside, when used in combination, can synergistically increase ARE activated gene transcription, synergistically restore glycolytic ATP production rates reduced by ROS-induced oxidative stress, and synergistically reduce ATP depletion caused by ROS. It is known that niacinamide and nicotinamide riboside are both NAD+ precursors, and thus capable of increasing NAD+ levels via known biological pathways, as shown in FIG. 2, but the synergy observed when these two materials are used in combination suggests they may also operate via unknown biological pathways, which is unexpected.

The improved cellular function provided by nicotinamide riboside and niacinamide may translate into a variety of overall skin health improvements such as improved skin tone (lighter and/or more even), reduced hyperpigmentation, reduced sallowness, improved skin barrier function (e.g., less trans-epidermal water loss), reduced fine lines and wrinkles, and improved texture (e.g., smaller pore size).

All ingredient percentages are by weight of the corresponding composition, unless otherwise specified. All ratios are weight ratios, unless specifically stated otherwise. All numeric ranges are inclusive of narrower ranges; delineated upper and lower range limits are interchangeable to create further ranges not explicitly delineated. The number of significant digits conveys neither limitation on the indicated amounts nor on the accuracy of the measurements. All measurements are understood to be made at about 25 °C and at ambient conditions, where "ambient conditions" means conditions under about one atmosphere of pressure and at about 50% relative humidity.

### Definitions.

"Additive effect" means the effect provided by a combination of niacinamide and nicotinamide riboside is equal to or approximately equal to the sum of their individual effects. For example, an additive effect would be demonstrated if nicotinamide riboside provided a 10% increase in ARE activated gene transcription when used alone, and niacinamide provided a 20% increase in ARE activated gene transcription when used alone, and together they provided about a 30% increase in ARE activated gene transcription (e.g., 28% to 32%). A "more than additive effect" (i.e., a synergistic effect) in this example would be demonstrated if the combination of nicotinamide riboside and niacinamide provided more than a 30% improvement in ARE activated gene transcription (e.g., 35%, 40%, 45%, 50% or more).

"Apply" or "application", as used in reference to a composition, means to apply or spread the compositions of the present invention onto a human skin surface.

"Cosmetic" means providing a desired visual effect on an area of the human body. The visual cosmetic effect may be temporary, semi-permanent, or permanent.

"Cosmetic agent" means any substance, as well any component thereof, intended to be rubbed, poured, sprinkled, sprayed, introduced into, or otherwise applied to a mammalian body or any part thereof to provide a cosmetic effect. Cosmetic agents may include substances that are Generally Recognized as Safe (GRAS) by the US Food and Drug Administration and food additives.

"Disposed" means an element is positioned in a particular place relative to another element.

"Effective amount" means an amount of nicotinamide riboside and niacinamide in combination that is sufficient to provide a desired benefit (e.g., increase in ARE activated gene transcription, restoration of glycolytic ATP production rates reduced by ROS-induced oxidative stress, reduction in ATP depletion caused by ROS) over the course of a treatment period.

"Oral Composition" means any composition in any form intended to be administered to the stomach of a human. For example, the oral composition may be provided in the form of a tablet (including a chewable tablet), pill, capsule, lozenge, troches, cachets, pellets, liquid, syrup, powder and the like.

"Stressor" mean an environmental element that causes the formation of reactive oxygen species in a cell. Non-limiting examples of stressors include ultraviolet radiation, cigarette smoke, ozone, engine exhaust, diesel exhaust, smog, surfactants, and radiation from a computer monitor or television.

"Safe and effective amount" means an effective amount of nicotinamide riboside and niacinamide in combination that is low enough to avoid serious side effects, within the scope of sound medical judgment.

"Skin" means the outermost protective covering of mammals that is composed of skin cells such as keratinocytes, fibroblasts and melanocytes. Skin includes an outer epidermal layer and an underlying dermal layer.

"Skin-care" means regulating and/or improving a skin condition. Some nonlimiting examples include improving skin appearance and/or feel by providing a smoother, more even appearance and/or feel; increasing the thickness of one or more layers of the skin; improving the elasticity or resiliency of the skin; improving the firmness of the skin; and reducing the oily, shiny, and/or dull appearance of skin, improving the hydration status or moisturization of the skin, improving the appearance of fine lines and/or wrinkles, improving skin exfoliation or desquamation, plumping the skin, improving skin barrier properties, improve skin tone, reducing the appearance of redness or skin blotches, and/or improving the brightness, radiancy, or translucency of skin. Some nonlimiting examples of "skin-care products" include skin creams, moisturizers, lotions, and body washes.

"Skin-care composition" means a composition that regulates and/or improves skin condition.

"Skin-care active" mean a compound or a combination of two or more compounds that, when applied to skin, provide an acute and/or chronic benefit to skin or a type of cell commonly found therein. Skin-care actives may regulate and/or improve skin or its associated cells (e.g., improve skin elasticity; improve skin hydration; improve skin condition; and improve cell metabolism).

"Synergy" and variations thereof mean an effect provided by two or materials (e.g., niacinamide and nicotinamide riboside) that is more than the additive effect expected for these materials. For example, synergy may be demonstrated when: (i) ARE activated gene expression provided by a combination of niacinamide and nicotinamide riboside is more than their calculated additive effect; (ii) restoration of a cell's glycolytic ATP production rate provided by a combination of niacinamide and nicotinamide riboside is more than their calculated additive effect and/or (iii) reduction in ATP depletion associated with oxidative stress caused by ROS provided by a combination of niacinamide and nicotinamide riboside is more than their calculated additive effect. ARE activated gene transcription and the effects of ROS on cellular metabolism and ATP depletion can be determined according to the methods described in more detail below.

"Treatment period," as used herein means the length of time and/or frequency that a material or composition is applied to a target skin surface.

The methods and compositions herein comprise a safe and effective amount of nicotinamide riboside (CAS No. 1341-23-7), which has the formula:

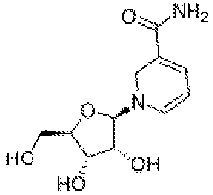

Examples of nicotinamide riboside and methods of making it are described in USPN 8,106,184. The methods and compositions herein also comprise a safe and effective amount of niacinamide (CAS No. 98-92-0), which has the formula:

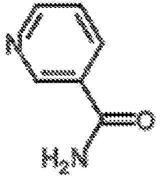

The compositions herein include nicotinamide riboside and niacinamide at an amount sufficient to increase ARE activated gene expression, restore glycolytic ATP production rates reduced by ROS-induced oxidative stress, and reduce ATP depletion caused by ROS, compared to untreated cells and/or according to one of the test methods described herein. Thus, it may be desirable to formulate the compositions herein to include an amount of nicotinamide riboside and niacinamide that increases ARE activated gene expression by at least 50 net luminescence (e.g., from 100 to 10,000, 200 to 7,500, 300 to 5,000 or any value in these ranges), according to the ARE Assay described below. Additionally or alternatively, it may be desirable to formulate the composition such that the combination of nicotinamide riboside and niacinamide restores glycolytic ATP production rates reduced by ROS-induced oxidative stress by at least 10% (e.g., 15%, 20%, 25%, 30%, 35%, 40%, 45% 50%, 555, 60%, 65%, 70%, 75%, 80%, 85%, 90%, 95% or even 100%), according to the ECAR Method described below. It may further be desirable to formulate the composition such that the combination of nicotinamide riboside and niacinamide reduces ATP depletion caused by ROS by at least 10% (e.g., 15%, 20%, 25%, 30%, 35%, 40%, 45% 50%, 555, 60%, 65%, 70%, 75%, 80%, 85%, 90%, 95% or even 100%), according to the ATP Assay described below.

While it can be important to provide a minimum level of ARE activation and metabolic stability to provide a desired cosmetic benefit, the compositions herein may also be formulated to include amounts of niacinamide and nicotinamide riboside that provide synergy for these benefits. For example, the amounts of niacinamide and nicotinamide riboside may be selected to increase ARE activated gene expression by at least 5% (e.g., 10%, 15%, 20%, 25%, 30%, 35%, 40%, 45% or even 50%) over the additive effect of these ingredients. Additionally or alternatively, the amounts of niacinamide and nicotinamide riboside may be selected to restore glycolytic ATP production rates reduced by ROS-induced oxidative stress by at least 5% (e.g., 10%, 15%, 20%, 25%, 30%, 35%, 40%, 45% or even 50%) over the additive effect of these ingredients on glycolyltic ATP restoration. In some instances, the amounts of niacinamide and nicotinamide riboside the may be selected to reduce ATP depletion caused by ROS by at least 5% (e.g., 10%, 15%, 20%, 25%, 30%, 35%, 40%, 45% or even 50%) over the additive effect of these ingredients to reduce ATP depletion.

Without intending to be bound by theory, it is believed that nicotinamide riboside may be primarily responsible for increasing ARE activated gene expression, as suggested from the results of testing nicotinamide riboside and niacinamide individually on their ability to modulate the ARE. Thus, it may be desirable to include the nicotinamide riboside and niacinamide in the present compositions at a particular ratio of NR:N (e.g., from 1:4 to 4:1, from 1:3 to 3:1, from 1:2 to 2:1, from 2:3 to 3:2 or even 1:1) to synergistically provide a suitable level of ARE activation in skin cells.

### Topical Cosmetic Compositions

In some instances, it may be desirable to use a topical cosmetic composition in accordance with the present methods. The topical cosmetic compositions herein may be provided in various product forms that include, for example, solutions, suspensions, lotions, creams, gels, toners, sticks, sprays, aerosols, ointments, cleansing liquid washes and solid bars, pastes, foams, mousses, shaving creams, wipes, strips, patches, electrically-powered patches, hydrogels, film-forming products, facial and skin masks (with and without insoluble sheet), make-up such as foundations, eye liners, and eye shadows, and the like. The cosmetic composition form may follow from the particular dermatologically acceptable carrier chosen.

Topical compositions suitable for use herein may be formed by combining effective amounts of niacinamide and nicotinamide riboside with a dermatologically acceptable carrier using known methods for making such compositions. It is to be appreciated that the amount of nicotinamide riboside in the present compositions may vary depending on how much of a particular benefit is desired. In some instances, the nicotinamide riboside and the niacinamide may each be present in the topical composition at the time of use and/or at the time of manufacture (i.e., added during formulation) at about 0.001% to 10% by weight based on the weight of the composition (e.g., from 0.005% to 9%, 0.01% to 8%, 0.1 % to 7%, 0.5% to 6%, 1% to 5%, 2% to 4%, or any value in these ranges).

It is known that nicotinamide riboside can undergo hydrolysis in an aqueous environment, depending on temperature and time in solution, to form niacinamide and ribose. This can be especially problematic for cosmetic compositions that are stored (e.g., in a warehouse, during shipping or on a store shelf) for a relatively long period of time (e.g., from 1 to 3 weeks or even from 1 to 6 months) at variety of temperatures. Typical storage temperatures for cosmetic compositions may range from 5° C to 45° C. Thus, in order to provide an effective amount of nicotinamide riboside in an aqueous composition (i.e., a composition that includes water), it is important to base the amount of nicotinamide riboside added during formulation on the known chemical kinetics of nicotinamide riboside and water. A discussion of the reaction kinetics of nicotinamide riboside and water can be found in a publication by Ferraz, et al., titled "Kinetic α-Deuterium Isotope Effects for Enzymatic and Nonenzymatic Hydrolysis of Nicotinamide-β-Riboside," Archives of Biochemistry and Biophysics Vol. 191, No. 2, December, pp. 431-436, 1978. Accordingly, the amounts of nicotinamide riboside disclosed herein may be present at the time of manufacture (i.e., during formulation) and/or at the time of use, depending on the desired level of benefit desired.

### Dermatologically Acceptable Carrier

The topical cosmetic compositions herein include a dermatologically acceptable carrier (which may be referred to as a "carrier"). The phrase "dermatologically acceptable carrier" means that the carrier is suitable for topical application to the keratinous tissue, has good aesthetic properties, is compatible with the actives in the composition, and will not cause any unreasonable safety or toxicity concerns. In one embodiment, the carrier is present at a level of from about 50% to about 99%, about 60% to about 98%, about 70% to about 98%, or, alternatively, from about 80% to about 95%, by weight of the composition.

The carrier can be in a wide variety of forms. In some instances, the solubility or dispersibility of the components may dictate the form and character of the carrier. Non-limiting examples include simple solutions (e.g., aqueous or anhydrous), dispersions, emulsions, and solid forms (e.g., gels, sticks, flowable solids, or amorphous materials). In certain embodiments, the dermatologically acceptable carrier is in the form of an emulsion. Emulsions may be generally classified as having a continuous aqueous phase (e.g., oil-in-water and water-in-oil-in-water) or a continuous oil phase (e.g., water-in-oil or oil-in-water). The oil phase of the present invention may comprise silicone oils, non-silicone oils such as hydrocarbon oils, esters, ethers, and the like, and mixtures thereof. The aqueous phase typically comprises water and water-soluble ingredients (e.g., water-soluble moisturizing agents, conditioning agents, anti-microbials, humectants and/or other skin care actives). However, in some instances, the aqueous phase may comprise components other than water, including but not limited to water-soluble moisturizing agents, conditioning agents, anti-microbials, humectants and/or other water-soluble skin care actives. In some instances, the non-water component of the composition comprises a humectant such as glycerin and/or other polyol(s). Emulsions may also contain an emulsifier, e.g., from about 1% to about 10% or from about 2% to about 5% based on the weight of the carrier. Emulsifiers may be nonionic, anionic or cationic. Some non-limiting examples of emulsifiers are disclosed in U.S. Pat. No. 3,755,560 to Dickert et al.; U.S. Pat. No. 4,421,769 to Dixon et al.; and McCutcheon's Detergents and Emulsifiers, North American Edition, pages 317-324 (1986).

The carrier may contain one or more dermatologically acceptable, hydrophilic diluents. As used herein, "diluent" includes materials in which the nicotinamide riboside and/or niacinamide can be dispersed, dissolved, or otherwise incorporated. Hydrophilic diluents include water, organic hydrophilic diluents such as lower monovalent alcohols (e.g., C1 -C4) and low molecular weight glycols and polyols, including propylene glycol, polyethylene glycol (e.g., Molecular Weight 200-600 g/mole), polypropylene glycol (e.g., Molecular Weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations of these.

### Additional Optional Ingredients

The cosmetic compositions herein may include one or more optional ingredients of the kind commonly included in the particular cosmetic compositing being provided (e.g., colorants, skin tone agents, skin anti-aging agents, anti-inflammatory agents, sunscreen agents, combinations of these and the like), provided that the additional ingredients do not undesirably alter the anti-oxidant benefits provided by the composition. When present, the optional ingredients may be included at amounts of from 0.0001% to 50%; from 0.001% to 20%; or even from 0.01% to 10% (e.g., 50%, 40%, 30%, 20%, 10%, 5%, 4%, 3%, 2%, 1%, 0.5% or 0.1%), by weight of the composition. The additional ingredients, when incorporated into the composition, should be suitable for use in contact with human skin tissue without undue toxicity, incompatibility, instability, allergic response, and the like. Some nonlimiting examples of additional ingredients which may be suitable for use herein are described in U.S. Publication Nos. 2002/0022040; 2003/0049212; 2004/0175347; 2006/0275237; 2007/0196344; 2008/0181956; 2010/00092408; 2008/0206373; 2010/0239510; 2010/0189669; 2011/0262025; 2011/0097286; US2012/0197016; 2012/0128683; 2012/0148515; 2012/0156146; and 2013/0022557; and U.S. Pat. Nos. 5,939,082; 5,872,112; 6,492,326; 6,696,049; 6,524,598; 5,972,359; and 6,174,533.

The topical cosmetic compositions herein may be generally prepared according to conventional methods known in the art of making such compositions. The methods typically involve mixing of ingredients in one or more steps to a relatively uniform state, with or without heating, cooling, application of vacuum, and the like. For example, emulsions may be prepared by first mixing the aqueous phase materials separately from the fatty phase materials and then combining the two phases as appropriate to yield the desired continuous phase. In certain embodiments, the compositions may be prepared to provide suitable stability (physical stability, chemical stability, photostability, etc.) and/or delivery of active materials. The composition may be provided in a package sized to store a sufficient amount of the composition for a treatment period.

### Method of Use for Topical Compositions

The methods herein include applying an effective amount of niacinamide and nicotinamide riboside to a target portion of skin at an amount and frequency sufficient to increase or synergistically increase ARE activated gene transcription, restore or synergistically glycolytic ATP production rates reduced by ROS-induced oxidative stress and/or reduce or synergistically reduce ATP depletion caused by ROS in at least some or all of the skin cells over the course of a treatment period. The target portion of skin may be identified by the consumer (e.g., self-diagnosis) and/or a skin care professional using, for example, visual evaluation techniques and/or digital imaging equipment. The target portion of skin is not particularly limited and may include any portion of skin that is exposed to environmental stressors and where an increase in ARE activated gene transcription and/or a reduction in ATP depletion caused by ROS is needed and/or desired. Such portions of skin tend to be those that are typically not covered by clothing (e.g., facial skin surfaces, hand and arm skin surfaces, foot and leg skin surfaces, and neck and chest skin surfaces). In some instances, the target portion of skin may be identified by exhibiting one or more signs of sign of skin aging, compromised skin health and/or skin cell dysfunction (e.g., hyperpigmented spot, fine lines, wrinkles, enlarged pores, undesirable texture, uneven skin tone, sallowness, dryness, reduced skin thickness, loss of elasticity). In some instances, the target portion of skin may not exhibit a sign of skin aging, compromised skin health and/or skin cell dysfunction, but a user (e.g., a relatively young user) may still wish to target this portion of skin, if it is one that typically develops such issues. In this way, the present methods and compositions may be used as a preventative measure.

The effective amount of nicotinamide riboside and niacinamide may be incorporated into a single topical cosmetic composition as described herein. Additionally or alternatively, two or more topical cosmetic compositions comprising nicotinamide riboside and/or niacinamide may be used as part of a regimen that delivers an effective amount of nicotinamide riboside and niacinamide when applied to a target portion of skin. For example, a first composition comprising nicotinamide riboside may be applied to a target skin surface, followed by application of a second composition comprising niacinamide to the target skin surface, or vice versa. In this example, the two compositions should be formulated such that sequential application of the compositions delivers an effective amount of nicotinamide riboside and niacinamide to the target portion of skin.

The treatment period is ideally of sufficient time for the combination of nicotinamide riboside and niacinamide to provide a synergistic increase in ARE activated gene transcription, a synergistic restoration of glycolytic ATP production rate reduced by ROS-induced oxidative stress and/or a synergistic reduction in ATP depletion caused by ROS. The treatment period lasts at least 1 week (e.g., about 2 weeks, 4 weeks, 8 weeks, or even 12 weeks). In some instances, the treatment period may extend over multiple months (*i.e.,* 3-12 months) or multiple years. In some instances, the composition may be applied most days of the week (e.g., at least 4, 5 or 6 days a week), at least once a day or even twice a day during a treatment period of at least 2 weeks, 4 weeks, 8 weeks, or 12 weeks.

The compositions herein may be applied locally or generally. In reference to application of the composition, the terms "localized", "local", or "locally" mean that the composition is delivered to the targeted portion of skin while minimizing delivery to portions of skin where treatment is not desired. The composition may be applied and lightly massaged into an area of skin. The form of the composition or the dermatologically acceptable carrier should be selected to facilitate localized application. While certain embodiments herein contemplate applying a composition locally to an area, it will be appreciated that compositions herein can be applied more generally or broadly to one or more skin surfaces. In certain embodiments, the compositions herein may be used as part of a multi-step beauty regimen, wherein the present composition may be applied before and/or after one or more other compositions.

### Oral Compositions

In some instances, it may be desirable to use an orally ingestible composition comprising an effective amount of nicotinamide riboside and niacinamide in accordance with the present methods. The unit dosage of an oral composition may comprise nicotinamide riboside and niacinamide at an amount of greater than 100 mg, or 200 mg, or 300 mg, or 400 mg, or 500 mg each. In some instances, the consumer may ingest a daily amount of nicotinamide riboside and/or niacinamide of greater than 100 mg, or 200 mg, or 300 mg, or 400 mg, or 500 mg, or 600 mg, but typically less than 1000 mg or 800 mg. One or more unit dosage forms of an oral composition may be ingested daily in order to achieve the desired daily effective amount over the course of a treatment period. It is to be appreciated that the amount of nicotinamide riboside and/or niacinamide included in the present compositions may vary depending on how much ARE activation and/or ATP restoration is desired.

### Other Optional Materials

The oral compositions may contain a wide variety of optional materials commonly included in oral compositions. For example, the oral composition may include a coating, which is typically applied to all or a part of a unit dosage form of an oral composition to improve the mouth feel, improve aesthetics and/or act as a barrier. When applied to the oral compositions herein, the coating may include a plasticizer such as polyethylene glycol or polypropylene glycol at 15% to 40% by weight of the coating material. In some instances, the oral composition may include a lubricating agent (e.g., talc, magnesium stearate, calcium stearate, stearic acid, hydrogenated vegetable oils, polyethylene glycol, sodium benzoate, sodium chloride, leucine, sodium lauryl sulfate, and magnesium lauryl sulfate) at an amount of less than 5% or even less than 1% by weight. The oral composition may include flavoring agents and/or sweeteners (e.g., natural and artificial flavoring agents and sweeteners, spray dried flavoring agents, encapsulated flavoring agents, water soluble sweeteners, water insoluble sweeteners, intense sweeteners and combinations of these). The amount of the flavoring agent and/or sweetener, when included, will vary as a matter of preference subject to such factors as flavor type and the strength of flavor or sweetness desired. Pigments, dyes, colorants and their corresponding lakes, which are suitable for human ingestion, may be added to modify the appearance of the oral composition to improve product aesthetics. The oral composition may include one or more nutrients (e.g., minerals, vitamins, oral nutritional supplements, enteral nutritional supplements, herbals and mixtures thereof). In some instances, the oral composition may include a binder (e.g., starches; gelatin; microcrystalline cellulose; polyvinyl pyrrolidone; cellulosics such as methyl cellulose, hydroxypropyl cellulose, hydroxypropylmethylcellulose, ethyl cellulose, and hydroxyethyl cellulose; other natural and synthetics gums such as carboxymethylcellulose, acacia, sodium alginate, and Veegum; and mixtures thereof).

### Methods of Use for Oral Compositions.

The present method includes identifying a target portion of skin where an increase or synergistically increase in ARE activated gene transcription, restoration or synergistic restoration of glycolytic ATP production rates reduced by ROS-induced oxidative stress and/or reduced or synergistically reduced ATP depletion caused by ROS is desired and/or needed, and orally ingesting a safe and effective amount of nicotinamide riboside and niacinamide in combination at an amount and frequency sufficient to provide the needed and/or desired benefit(s) over the course of a treatment period. The target portion of skin may be identified by the consumer (e.g., self-diagnosis) and/or a skin care professional using, for example, visual evaluation techniques and/or digital imaging equipment. The target portion of skin is not particularly limited and may include any portion of skin that is exposed to environmental stressors and/or where an increase in ARE activated gene transcription and/or a reduction in ATP depletion caused by ROS is desired and/or needed. Such portions of skin tend to be disposed on areas of the body that are typically not covered by clothing (e.g., facial skin surfaces, hand and arm skin surfaces, foot and leg skin surfaces, and neck and chest skin surfaces). In some instances, the target portion of skin may be identified by exhibiting one or more signs of skin aging, compromised skin health and/or skin cell dysfunction (e.g., hyperpigmented spot, fine lines, wrinkles, enlarged pores, undesirable texture, uneven skin tone, sallowness, dryness, reduced skin thickness, loss of elasticity). In some instances, the target portion of skin may not exhibit a sign of skin aging, compromised skin health and/or skin cell dysfunction, but a user (e.g., a relatively young user) may still wish to target such an area of skin, if it is one that typically develops such issues. In this way, the present method may be used as a preventative measure for skin health disorders.

The effective amount of nicotinamide riboside and niacinamide may be incorporated into a single oral composition as described herein. Additionally or alternatively, two or more oral compositions comprising nicotinamide riboside and/or niacinamide may be used as part of a regimen that delivers an effective amount of nicotinamide riboside and niacinamide when ingested. For example, a first oral composition comprising nicotinamide riboside may be ingested, followed by oral ingestion of a second composition comprising niacinamide, or vice versa. In this example, the sequential oral ingestion of the two compositions should deliver an effective amount of nicotinamide riboside and niacinamide. In some instances, the oral composition may be used in combination with and/or as a substitute for a topical composition comprising an effective amount of nicotinamide riboside and niacinamide.

The oral composition may be ingested one time per day, two times per day, three times per day (e.g., around each meal), four times per day or more during the treatment period. The daily dosage of nicotinamide riboside may be greater than 100 mg, greater than 250 mg, greater than 300 mg, or even greater than 400 mg or 500 mg. The weekly dosage may be greater than 2100 mg/week, 2800 mg/week, or 3500 mg/week. The daily dosage may be provided in a single unit dosage form (e.g., a single pill, capsule or tablet) or may be provided in smaller unit dosage forms if the oral composition is intended to be taken more than once per day.

The treatment period is ideally of sufficient time for the combination of nicotinamide riboside and niacinamide to provide a synergistic increase in ARE activated gene transcription and/or a synergistic reduction in ATP depletion caused by ROS. For example, the treatment period may last at least 3 weeks, 4 weeks, 6 weeks, or even at least 8 weeks. In some instances, the treatment period may extend over multiple months (e.g., 1, 2, 3, 4, 5, 6, 7, 8, 10, 12 or more months) or multiple years (e.g., 1, 2, 3, 4, 5 or more years). Without intending to be bound by theory, it is believed within 2 weeks or less after cessation of the treatment period, there may be a regression to baseline in the one or more of the benefits provided by the compositions and methods herein. Thus, it may be desirable for the daily dosage to be uninterrupted during the treatment period, or interrupted for less than 2 weeks, or less than 1 week, or less than 5 days, or less than 3 days.

### EXAMPLES

### Example 1 - Synergistic activation of the Antioxidant Response Element

This example demonstrates the ability of an effective amount of a combination of niacinamide and nicotinamide riboside to synergistically activate the ARE. ARE activation was quantitated using the ARE-32 reporter cell line available from CXR-Biosciences as described in the ARE Assay below.

### ARE Assay.

ARE-32 is a stable MCF7 cell line containing pGL8x-ARE (8 copies of the rat GST ARE linked to the luciferase gene) and pCDNA3.1, which contains the neomycin selectable marker. Selection was performed in the presence of G418 and resistant clones were isolated. Clones were screened for induction of luciferase in response to tBHQ.

Reagents and Instruments used in this example are provided below.

Dulbecco's Modified Eagle Medium (DMEM) (Gibco, Cat #11054-020, lot#1361242)
Fetal Bovine Serum Heat Inactivated (FBS) (Gibco, Cat # 16140-063, lot#1345764)
Geneticin G418 sulphate (G418) (Gibco, Cat # 11811-031)
Steady Glo System (Promega, Cat # E2510)
96-well plate (Costar, Cat # 3917)
Penicillin-Streptomycin (Gibco, Cat # 15070-063, Lot # 109733)
Tert-Butylhydroquinone (tBHQ) (Aldrich, Cat #11, 294-1)

### Perkin Elmer Envision reader

It is to be appreciated that equivalent reagents and instruments may be substituted for those shown, as long as the substitution would not be expected to alter the results of the assay.

### Maintenance of ARE-32 Cells

The ARE-32 cells are maintained routinely in the DMEM (phenol red free) containing: 10% FBS, 50 units/ml penicillin & 50 µg/ml streptomycin, 0.8 mg/ml G418. Cells are subcultured every 3-4 days. Cells were frozen in medium that contains 90% FBS and 10% DMSO.

Induction of Luciferase with treatments and tBHQ as a positive control.

In a 96 well-plate, seed 1x10⁴ cells/well in 100 µl DMEM containing 50 units/ml penicillin, 50 µg/ml streptomycin, 0.8 mg/ml G418 and 10% FBS. Next, incubate the cells at 37° C. in a 5% CO₂ incubator for 24 hrs, and then replace the medium with 100 µl fresh media. Treat with test compounds at 2 ul per well, positive control was 25 uM TBHQ. (10 mM tBHQ of stock solution freshly prepared in DMSO). Add 100 ul of media after treatment for a final assay volume of 200 uL. Incubate the cells at 37° C in the CO₂ incubator for another 24 hrs. Assay for luciferase activity with a STEADY-GLO brand assay system according to the manufacturer's instructions. A general schematic for how the ARE reporter assay operates to identify agents that promote transcription off the ARE is described in U.S. Publication No. 2011/0262570.

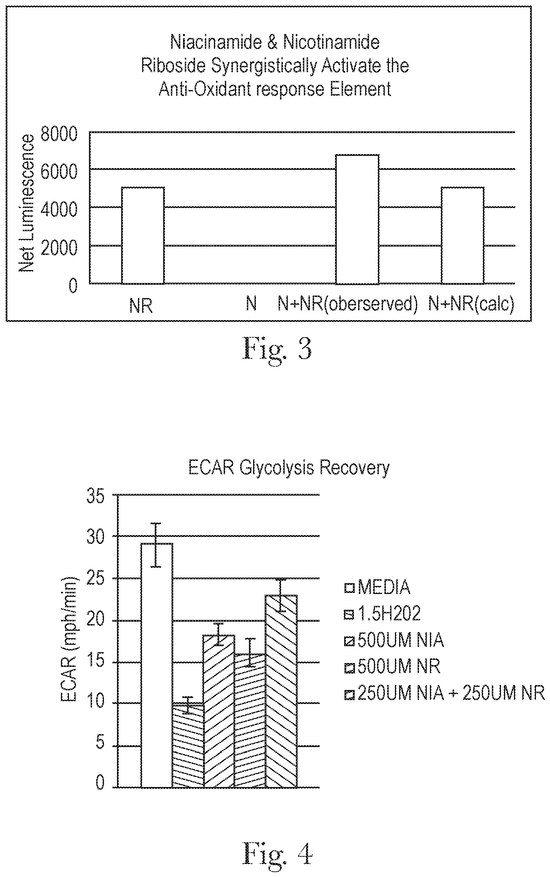

The results of the test are illustrated in Table 1 and FIG. 3. The ARE activity for nicotinamide ("NR"), niacinamide ("N") and the combination of nicotinamide riboside and niacinamide is reported as net luminescence in Table 1. Net luminescence for a test sample is determined by subtracting the luminescence value of the control from the luminescence value of the test sample. The calculated net luminescence for NR + N is the sum of the individual measured luminescence values for NR and N.

As shown in Table 1, the nicotinamide riboside provided higher luminescence than niacinamide, suggesting that niacinamide and nicotinamide riboside function via different biological pathways and niacinamide alone may not be suitable for use as an ARE activator. But surprisingly, the combination of niacinamide and nicotinamide riboside appears to increase ARE activated gene transcription synergistically.

**Table 1**

| | | Net Luminescence | | | | |
|---|---|---|---|---|---|---|
| NR (w/v%) | N (w/v%) | NR | N | NR+N (observed) | NR+N (calculated) | p-value (observed vs calc) |
| 0.0842 | 0.1 | 5176 | 75 | 7982 | 5251 | 8.92 x 10⁻⁵ |
| 0.042 | 0.05 | 1845 | 71 | 3360 | 1916 | 0.0487 |
| 0.021 | 0.025 | 594 | 26 | 1458 | 620 | 0.0071 |
| 0.011 | 0.0125 | 205 | 13 | 551 | 217 | 0.1060 |

### Example 2 - Synergistic restoration of glycolytic ATP production rate reduced by ROS-induced oxidative stress using the ECAR Method.

This example demonstrates the ability of a combination of nicotinamide riboside and niacinamide to synergistically restore glycolytic ATP production rate reduced by ROS-induced oxidative stress. Glycolytic ATP production by fibroblasts exposed to hydrogen peroxide was determined using an XF Extracellular Flux brand analyzer available from Seahorse Bioscience, Massachusetts The analyzer measured extracellular acidification rate ("ECAR") in real time, which correlates to glycolytic ATP production.

### ECAR Method

The cells used in this test were frozen human dermal fibroblasts obtained from the BJ cell line commercially available from ATCC, Bethesda, MD. The fibroblasts were grown to 70-80% confluence in a culture medium of Eagle's Minimum Essential Medium ("EMEM") supplemented with 10% fetal bovine serum ("FBS") and gentamicin/amphotericin B x500 solution (EMEM and FBS are available from ATCC, and gentamicin/amphotericin B x500 solution is available from Invitrogen). The fibroblasts were plated at 8x10⁴ cells per well in gelatin-coated plates 24 hours prior to testing. Each well included 100 µL of the culture medium described above.

The gelatin-coated plates are prepared by coating each well in a multi-well plate (e.g., 24-well or 96-well plates available from Seahorse Biosciences, Massachusetts) with 0.2% gelatin (protein solution available from Sigma) diluted with phosphate buffered saline ("PBS"). The gelatin is diluted 1:10 to 0.2% in sterile PBS at 37° C, and then 50 uL of the diluted gelatin solution is added to each well and incubated at room temperature for 30 minutes. Excess liquid is removed by aspiration without touching the wells and the surface is allowed to dry for at least 2 hours.

Hydrogen peroxide is a well-known ROS, and is used in this example as a source of oxidative stress on the fibroblasts being tested. A stressor solution of hydrogen peroxide was prepared at 10X working concentration in the fibroblast test medium described above. The test agents (i.e., nicotinamide riboside, niacinamide, and nicotinamide riboside + niacinamide in combination) were prepared at 10X the final working concentration in the fibroblast test medium to provide test agent solutions. The stressor solution and the test agent solutions were warmed to 37° C and pH adjusted to 7.4. A fibroblast test medium was made from Dulbecco's Modified Eagle Medium (available from Seahorse Bioscience), 25mM glucose and 1mM pyruvate. The fibroblast test medium was warmed to 37° C and the pH adjusted to 7.4. The cells were grown and plated at 37° C and 5% CO₂ air. The culture medium in the wells was replaced by the fibroblast test medium 1 hour prior to taking measurements. Each well contained approximately 8x10⁴ cells and 600 µL of fibroblast test medium (i.e., DMEM, 25 mM glucose and 1 mM pyruvate) at 37° C and pH 7.4.

The plates containing the samples to be tested were loaded into the analyzer and equilibrated according to the manufacturer's instructions. The test agent solutions and stressor solution were each placed in an automated injection port of the XF cartridge plate. The analyzer was programmed to sequentially run a two minute mix cycle, a two minute wait cycle, and a 3 minute measurement cycle continuously for at least 88 minutes. Data points were collected and recorded by the analyzer. The analyzer was allowed to complete three cycles prior to the addition of a stressor solution or test agent solution to provide a basal ECAR value. After the third cycle was completed (i.e., about 21 minutes after detection began), the test solution and/or stressor solution was added to the wells by the analyzer from the appropriate injection port. A stressor solution was added to the well in sufficient amount to provide 1.5 mM hydrogen peroxide in the well. The test agent solution was added in sufficient amount to provide 250 µM or 500 µM niacinamide or nicotinamide riboside in the well.

The results of the test are illustrated in Table 2 and FIG. 4, nicotinamide riboside ("NR") and niacinamide ("N") appear to provide about the same level of protection again glycolytic rate alterations in the presence of hydrogen peroxide. Thus, testing a combination of NR and N at half the concentration at which each was tested individually was expected to yield an equivalent effect. In other words, 250 µM N + 250 µM NR should provide the same effect as the 500 µM N and 500 µM NR provided individually. But surprisingly, the combination of niacinamide and nicotinamide riboside appears to restore glycolytic ATP production synergistically. As illustrated in Table 2, the glycolytic ATP production restored by the combination of nicotinamide riboside and niacinamide was over 30% more than the expected additive amount (i.e., calculated amount) of the individual effects.

**Table 2**

| | Observed ECAR (mpH/min) | Standard Deviation | % Change from Control | Calculated ECAR for N + NR | % Change Observed vs. Calculated |
|---|---|---|---|---|---|
| Media - Control | 29.89 | 3.04 | - | - | - |
| Hydrogen Peroxide | 8.96 | 1.60 | -70.0% | - | - |
| N (500 µM) | 15.35 | 1.22 | -48.6% | - | - |
| NR (500 µM) | 14.31 | 1.19 | -52.1% | - | - |
| N + NR (250 µM each) | 19.41 | 1.60 | -35.1% | 14.83 | +30.9% |

### Example 3 - Synergistic reduction in ATP depletion caused by ROS using an ATP Assay.

This example demonstrates the ability of a combination of niacinamide and nicotinamide riboside to synergistically reduce ATP depletion caused by hydrogen peroxide, which is a well-known ROS commonly used to analyze the effects of ROS and oxidative stress on various cellular functions (e.g., metabolism). In this test, keratinocytes were exposed to various combinations of hydrogen peroxide, nicotinamide riboside and/or niacinamide to observe the effects of niacinamide and nicotinamide riboside on ATP depletion caused by hydrogen peroxide.

### ATP Assay

The keratinocytes were cultured in T150 flasks using EPILIFE brand culture medium (calcium free and phenol red free, supplemented with penicillin/streptomycin and keratinocytes growth supplement, Invitrogen cat# MEPICFPRF500). The keratinocytes were then plated in 24 well plates with 40,000 cells/well and 1 ml of culture medium. After 24 hours, the keratinocytes were treated with 500 uM hydrogen peroxide alone, 400 uM niacinamide, 400 uM nicotinamide riboside or a combination of niacinamide and nicotinamide riboside (400 uM each) for 1 hour. Untreated keratinocytes were used as a control (i.e., keratinocytes in culture medium). The keratinocytes were washed in PBS and ATP levels were measured using the Cell Titer-Glo® brand ATP assay available from Promega, Madison, WI as catalogue # G7571/2/3 according to the manufacturer's instructions. Luminescence was measured on a SPECTRAMAX M3 brand microplate reader (Molecular Devices, Sunnyvale, CA). The net luminescence for a test sample is calculated by subtracting the observed luminescence value for the control from the observed luminescence value for the test sample.

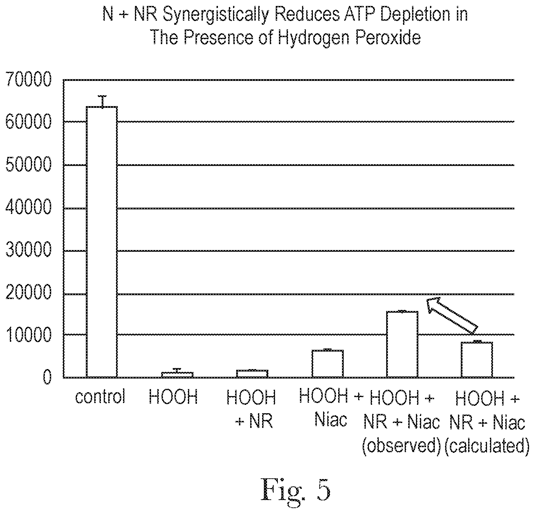

The results of the test are illustrated in Table 3 and FIG. 5. As illustrated in Table 3 and FIG. 5, hydrogen peroxide caused about a 97.5% depletion in ATP. The addition of the niacinamide and nicotinamide riboside with hydrogen peroxide resulted in less ATP depletion (about 89.7% and 96.9%, respectively). Surprisingly, when combination of niacinamide and nicotinamide was added to the hydrogen peroxide, ATP depletion was reduced by almost twice the amount that would be expected by their additive effect (i.e., observed net luminescence of 15525 vs. an expected net luminescence of 8510.5).

**Table 3**

| | Net Luminescence | | | | | |
|---|---|---|---|---|---|---|
| | control | H₂O₂ | H₂O₂ + NR | H₂O₂ + N | Observed H₂O₂+ NR + N | Calculated H₂O₂+ NR + N |
| AVG | 63548 | 1556 | 1971.5 | 6539 | 15525 | 8510.5 |
| StDev | 2394 | 885 | 147 | 300 | 275 | - |
| % depletion vs. control | - | 97.5% | 96.9% | 89.7% | 75.6% | 86.6% |
| p-value observed vs. calculated | - | - | - | - | 1. 116x10⁻⁷ | - |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm." Additionally, properties described herein may include one or more ranges of values. It is to be understood that these ranges include every value within the range, even though the individual values in the range may not be expressly disclosed.

## Claims

1. A cosmetic, non-therapeutic method of providing an anti-oxidant benefit to skin by activating gene transcription through the antioxidant response element (ARE) in a skin cell, comprising:
i. identifying a target portion of skin comprising skin cells where an anti-oxidant benefit is desired; and
ii. contacting at least some of the skin cells with an effective amount of niacinamide and nicotinamide riboside in combination for a treatment period sufficient for the niacinamide and nicotinamide riboside to increase ARE activated gene transcription in at least some of the contacted skin cells.

2. The method of claim 1, wherein ARE activated gene transcription is increased by at least about 10%, preferably at least about 20%, more preferably at least about 30% and most preferably at least about 50%, measured as per the method disclosed herein.

3. The method of claim 1 or 2, wherein the effective amount of niacinamide and nicotinamide riboside synergistically increases ARE activated gene transcription by at least 5%, preferably at least 10% more preferably at least 20%, and most preferable at least 30% more than an additive effect.

4. The method of any preceding claim, wherein the effective amount of niacinamide and nicotinamide riboside reduces ATP depletion caused by a reactive oxygen species (ROS).

5. The method of claim 4, wherein the effective amount of niacinamide and nicotinamide riboside synergistically reduces ATP depletion caused by the ROS.

6. The method of claim 5, wherein the synergistic reduction in ATP depletion is at least 5%, preferably at least 10% more preferably at least 20%, and most preferable at least 30% more than an additive effect.

7. The method of any preceding claim, wherein the effective amount of niacinamide and nicotinamide riboside synergistically restores glycolytic ATP production rate reduced by ROS by at least 5%, preferably at least 10% more preferably at least 20%, and most preferable at least 30% more than an additive effect, measured as per the method disclosed herein.

8. The method of any preceding claim, wherein the target portion of skin comprises facial skin.

9. The method of any preceding claim, wherein the treatment period is at least 2 weeks, preferably at least four weeks and more preferably at least 8 weeks.

10. The method of claim 8, wherein the treatment period is at least 4 weeks, and the effective amount of niacinamide and nicotinamide riboside provides a synergistic improvement in trans-epidermal water loss.

11. The method of any preceding claim, wherein the effective amount of niacinamide and nicotinamide riboside is contacted with the target skin portion by at least one of topical application and oral ingestion.

12. The method of any preceding claim, wherein the nicotinamide riboside and niacinamide are provided in a ratio of nicotinamide riboside to niacinamide of about 1:4 to about 4:1.

13. The method of any preceding claim, wherein the nicotinamide riboside and niacinamide are provided in a cosmetic composition comprising a dermatologically acceptable carrier.

## Patentansprüche

1. Kosmetisches, nicht therapeutisches Verfahren zur Bereitstellung eines Antioxidans-Nutzens für die Haut durch Aktivieren einer Gen-Transkription durch das Antioxidansreaktionselement (ARE) in einer Hautzelle, das Folgendes umfasst:
i. das Identifizieren eines Zielabschnitts der Haut, der Hautzellen umfasst, in denen ein Antioxidans-Nutzen erwünscht ist; und
ii. das Inkontaktbringen von mindestens einigen der Hautzellen mit einer wirksamen Menge an Niacinamid und Nicotinamidribosid in Kombination über eine Behandlungsdauer hinweg, die ausreicht, damit das Niacinamid und Nicotinamidribosid die ARE-aktivierte Gentranskription in zumindest einigen der kontaktierten Hautzellen erhöhen.

2. Verfahren nach Anspruch 1, wobei die ARE-aktivierte Gentranskription um mindestens etwa 10 %, vorzugsweise mindestens etwa 20 %, mehr bevorzugt mindestens etwa 30 % und am meisten bevorzugt mindestens etwa 50 % erhöht wird, gemessen gemäß dem hierin offenbarten Verfahren.

3. Verfahren von Anspruch 1 oder 2, wobei die wirksame Menge von Niacinamid und Nicotinamidribosid synergistisch die ARE-aktivierte Gentranskription um mindestens 5 %, vorzugsweise mindestens 10 %, besonders bevorzugt mindestens 20 % und am meisten bevorzugt mindestens 30 % mehr als einen additiven Effekt erhöht.

4. Verfahren nach einem der vorhergehenden Ansprüche, wobei die wirksame Menge an Niacinamid und Nicotinamidribosid die ATP-Verarmung reduziert, die durch eine reaktive Sauerstoffspezies (ROS) verursacht wird.

5. Verwendung nach Anspruch 4, wobei die wirksame Menge an Niacinamid und Nicotinamidribosid die ATP-Verarmung, die durch das ROS verursacht wird, synergistisch reduziert.

6. Verfahren nach Anspruch 5, wobei die synergistische Verringerung der ATP-Depletion mindestens 5 %, vorzugsweise mindestens 10 %, mehr bevorzugt mindestens 20 % und am am meisten bevorzugtest mindestens 30 % mehr als ein additiver Effekt ist.

7. Verfahren nach einem der vorhergehenden Ansprüche, wobei die wirksame Menge an Niacinamid und Nicotinamidribosid die glykolytische ATP-Produktionsrate, die durch ROS reduziert wurde, um mindestens 5 %, vorzugsweise mindestens 10 %, mehr bevorzugt mindestens 20 % und am meisten bevorzugt mindestens 30 % mehr als eine additive Wirkung synergistisch wiederherstellt, gemessen nach dem hier offenbarten Verfahren.

8. Verfahren nach einem der vorangehenden Ansprüche, wobei der Zielabschnitt der Haut Gesichtshaut umfasst.

9. Verfahren nach einem der vorangehenden Ansprüche, wobei die Behandlungsdauer mindestens 2 Wochen, vorzugsweise mindestens vier Wochen und mehr bevorzugt mindestens 8 Wochen beträgt.

10. Verfahren von Anspruch 8, wobei die Behandlungsdauer mindestens 4 Wochen beträgt und die wirksame Menge an Niacinamid und Nicotinamidribosid eine synergistische Verbesserung des transepidermalen Wasserverlusts bereitstellt.

11. Verfahren nach einem der vorstehenden Ansprüche, wobei die wirksame Menge an Niacinamid und Nicotinamid-Ribosid mit dem Zielhautabschnitt durch mindestens eines von topischer Anwendung und oraler Aufnahme in Kontakt gebracht wird.

12. Verfahren nach einem der vorhergehenden Ansprüche, wobei das Nicotinamidribosid und Niacinamid in einem Verhältnis von Nicotinamidribosid zu Niacinamid von etwa 1:4 bis etwa 4:1 bereitgestellt werden.

13. Verfahren nach einem der vorhergehenden Ansprüche, wobei das Nicotinamidribosid und Niacinamid in einer kosmetischen Zusammensetzung bereitgestellt werden, die einen dermatologisch verträglichen Träger umfasst.

## Revendications

1. Procédé cosmétique non thérapeutique de fourniture d'un effet bénéfique antioxydant à la peau par activation d'une transcription de gène par l'intermédiaire de l'élément de réponse antioxydante (ARE) dans une cellule de peau, comprenant :
i. l'identification d'une partie cible de peau comprenant des cellules de peau où un effet bénéfique antioxydant est souhaité ; et
ii. la mise en contact d'au moins certaines des cellules de peau avec une quantité efficace de niacinamide et de nicotinamide riboside en combinaison pendant une période de traitement suffisante pour permettre au niacinamide et au nicotinamide riboside d'augmenter la transcription de gène activée par ARE dans au moins certaines des cellules de peau mises en contact.

2. Procédé selon la revendication 1, dans lequel la transcription de gène activée par ARE est augmentée d'au moins environ 10 %, de préférence d'au moins environ 20 %, plus préférablement d'au moins environ 30 % et le plus préférablement d'au moins environ 50 %, mesurée selon le procédé décrit ici.

3. Procédé selon la revendication 1 ou 2, dans lequel la quantité efficace de niacinamide et de nicotinamide riboside augmente synergiquement la transcription de gène activée par ARE au moins 5 %, de préférence au moins 10 % plus préférablement au moins 20 %, et le plus préférablement au moins 30 % en plus qu'un effet additif.

4. Procédé selon l'une quelconque des revendications précédentes, dans lequel la quantité efficace de niacinamide et de nicotinamide riboside réduit la déplétion d'ATP provoquée par une espèce réactive d'oxygène (ROS).

5. Procédé selon la revendication 4, dans lequel la quantité efficace de niacinamide et de nicotinamide riboside réduit synergiquement la déplétion d'ATP provoquée par la ROS.

6. Procédé selon la revendication 5, dans lequel la réduction synergique de déplétion d'ATP est au moins 5 %, de préférence au moins 10 % plus préférablement au moins 20 %, et le plus préférablement au moins 30 % supérieure à un effet additif.

7. Procédé selon l'une quelconque des revendications précédentes, dans lequel la quantité efficace de niacinamide et de nicotinamide riboside restaure synergiquement le taux de production d'ATP glycolytique réduit par la ROS au moins 5 %, de préférence au moins 10 % plus préférablement au moins 20 %, et le plus préférablement au moins 30 % en plus qu'un effet additif, mesuré selon le procédé décrit ici.

8. Procédé selon l'une quelconque des revendications précédentes, dans lequel la partie cible de peau comprend la peau du visage.

9. Procédé selon l'une quelconque des revendications précédentes, dans lequel la période de traitement est d'au moins 2 semaines, de préférence au moins quatre semaines et plus préférablement au moins 8 semaines.

10. Procédé selon la revendication 8, dans lequel la période de traitement est d'au moins 4 semaines, et la quantité efficace de niacinamide et de nicotinamide riboside fournit une amélioration synergique de perte d'eau trans-épidermique.

11. Procédé selon l'une quelconque des revendications précédentes, dans lequel la quantité efficace de niacinamide et de nicotinamide riboside est mise en contact avec la partie de peau cible par au moins l'une parmi une application locale et une ingestion orale.

12. Procédé selon l'une quelconque des revendications précédentes, dans lequel le nicotinamide riboside et le niacinamide sont fournis dans un rapport du nicotinamide riboside au niacinamide d'environ 1:4 à environ 4:1.

13. Procédé selon l'une quelconque des revendications précédentes, dans lequel le nicotinamide riboside et le niacinamide sont fournis dans une composition cosmétique comprenant un véhicule acceptable du point de vue dermatologique.
